# EUROPEAN PATENT APPLICATION

(11) **EP 4 699 794 A1**
(43) Date of publication of application: **25.02.2026**
(21) Application number: 25196378.1
(22) Date of filing: 18.08.2025
(51) Int. Cl.: B29D 11/00, B65B 25/00

(54) **FORMING METHOD OF CONTACT LENS, CONTACT LENS ASSEMBLY, AND CONTACT LENS PRODUCT**

(30) Priority: 20.08.2024 US 202463684867 P
(71) Applicant: Pegavision Corporation, Taoyuan City 333 (TW)
(72) Inventor: CHANG, Han-Yi, 333 TAOYUAN CITY (TW); CHEN, Chun-Han, 333 TAOYUAN CITY (TW); HUANG, Wen-Chia, 333 TAOYUAN CITY (TW); LIN, Shih-Siang, 333 TAOYUAN CITY (TW); LIU, Li-Hao, 333 TAOYUAN CITY (TW); WU, Cheng-Hsuan, 333 TAOYUAN CITY (TW)
(74) Representative: Viering, Jentschura & Partner mbB Patent- und Rechtsanwälte

(57) **Abstract**

A contact lens assembly (100) includes a cup (11), a dry contact lens (12a, 12b) that is not for being worn on an eye (E), a sealing layer (13) removably connected to the cup (11), and a wetting liquid supply (2). The sealing layer (13) and the cup (11) jointly form an enclosed space (14) that receives the dry contact lens (12a, 12b) therein and that does not accommodate any liquid. After at least one of the sealing layer (13) and the cup (11) produces an opening (15), the cup (11) can receive a wetting liquid (21) from the wetting liquid supply (2) through the opening (15), such that the dry contact lens (12a, 12b) is immersed in the wetting liquid (21) to form a wet contact lens (3) that is suitable for being worn on the eye (E) and that has a water content greater than a water content of the dry contact lens (12a, 12b).

## Description

### FIELD OF THE INVENTION

The present invention relates to a contact lens, and more particularly to a forming method of contact lens, a contact lens assembly, and a contact lens product.

### BACKGROUND OF THE INVENTION

A conventional contact lens is sealed in a space that is formed by a cup-shaped structure and a sealing layer, and that is filled with a contact lens solution, but the development of the conventional contact lens is limited by the above configuration.

### SUMMARY OF THE INVENTION

In response to the above-referenced technical inadequacies, the present invention provides a forming method of contact lens, a contact lens assembly, and a contact lens product for effectively improving on the issues associated with conventional contact lenses.

In order to solve the above-mentioned problems, one of the technical aspects adopted by the present invention is to provide a forming method of a contact lens, which includes a preparation step, a sterilization step, and a wetting step. The preparation step is implemented by placing a dry contact lens that has a first water content into a cup after a drying process, and then using a sealing layer to seal the cup for jointly forming an enclosed space that receives the dry contact lens and that does not accommodate any liquid. The sterilization step is implemented by performing a dry contact lens sterilization process for the dry contact lens that is in the enclosed space. The wetting step is implemented by tearing away the sealing layer relative to the cup to form an opening, and then injecting a wetting liquid into the cup through the opening. After the dry contact lens is immersed in the wetting liquid for a predetermined period of time, the dry contact lens becomes a wet contact lens that is configured to be worn on a human eye and that has a second water content greater than the first water content.

In order to solve the above-mentioned problems, another one of the technical aspects adopted by the present invention is to provide a contact lens assembly, which includes a contact lens product and a wetting liquid supply. The contact lens product includes a cup, a dry contact lens, and a sealing layer. The dry contact lens is disposed in the cup and has a first water content. The dry contact lens has a shape that does not allow the dry contact lens to be worn on a human eye. The sealing layer is removably connected to the cup to jointly define an enclosed space. The enclosed space receives the dry contact lens and does not accommodate any liquid. The wetting liquid supply has a wetting liquid that is configured to wet the dry contact lens. When at least one of the sealing layer and the cup of the contact lens product produces an opening, the cup is configured to receive the wetting liquid from the wetting liquid supply through the opening, so that the dry contact lens is immersed in the wetting liquid to become a wet contact lens that has no cracks and that is configured to be worn on the human eye. The wet contact lens has a second water content being greater than the first water content.

In order to solve the above-mentioned problems, yet another one of the technical aspects adopted by the present invention is to provide a contact lens product, which includes a cup, a dry contact lens, and a sealing layer. The dry contact lens is disposed in the cup and has a first water content that is within a range from 0.001% to 20%. The dry contact lens has a shape that does not allow the dry contact lens to be worn on a human eye. The sealing layer is removably connected to the cup to jointly define an enclosed space. The enclosed space receives the dry contact lens and does not accommodate any liquid.

Therefore, any one of the forming method, the contact lens assembly, and the contact lens product in the present invention is provided with a configuration (e.g., the dry contact lens being arranged in the enclosed space defined by the cup and the sealing layer) different from that of the conventional contact lens product, thereby allowing the scope of development for contact lenses to be expanded (e.g., allowing the dry contact lens and the wetting liquid to be sterilized independently to improve the overall performance).

Specifically, in any one of the forming method and the contact lens assembly provided by the present invention, the dry contact lens and the wetting liquid are sterilized by respectively performing independent sterilization processes, such that under the premise of meeting their respective sterilization requirements, the dry contact lens and the wetting liquid can effectively retain optimal performance.

These and other aspects of the present invention will become apparent from the following description of the embodiment taken in conjunction with the following drawings and their captions, although variations and modifications therein may be effected without departing from the scope of the novel concepts of the invention.

### BRIEF DESCRIPTION OF THE DRAWINGS

The described embodiments may be better understood by reference to the following description and the accompanying drawings, in which:
FIG. 1 is a schematic perspective view of a contact lens assembly according to one embodiment of the present invention;
FIG. 2 is a schematic exploded view of FIG. 1;
FIG. 3 is a schematic perspective view showing one of contact lenses of FIG. 2;
FIG. 4 is a schematic cross-sectional view taken along line IV-IV of FIG. 1;
FIG. 5 is a schematic perspective view showing cups of the contact lens assembly of FIG. 1 that are injected with wetting liquid;
FIG. 6 is a schematic perspective view showing dry contact lenses of the contact lens assembly of FIG. 5 that are immersed in the wetting liquid;
FIG. 7 is a schematic perspective view showing the dry contact lenses of FIG. 6 that becomes wet contact lenses;
FIG. 8 is a schematic view showing the wet contact lenses of FIG. 7 that are worn on human eyes;
FIG. 9 is a schematic perspective view showing the contact lens assembly in another configuration for being injected with the wetting liquid;
FIG. 10 is a flowchart of a forming method of contact lens according to one embodiment of the present invention;
FIG. 11 is a schematic view showing a sterilization step of FIG. 10; and
FIG. 12 is a schematic view showing a sterile filtration process of the forming method according to the embodiment of the present invention.

### DETAILED DESCRIPTION OF THE EXEMPLARY EMBODIMENTS

The present invention is more particularly described in the following examples that are intended as illustrative only since numerous modifications and variations therein will be apparent to those skilled in the art. Like numbers in the drawings indicate like components throughout the views. As used in the description herein and throughout the claims that follow, unless the context clearly dictates otherwise, the meaning of "a," "an" and "the" includes plural reference, and the meaning of "in" includes "in" and "on." Titles or subtitles can be used herein for the convenience of a reader, which shall have no influence on the scope of the present invention.

The terms used herein generally have their ordinary meanings in the art. In the case of conflict, the present document, including any definitions given herein, will prevail. The same thing can be expressed in more than one way. Alternative language and synonyms can be used for any term(s) discussed herein, and no special significance is to be placed upon whether a term is elaborated or discussed herein. A recital of one or more synonyms does not exclude the use of other synonyms. The use of examples anywhere in this specification including examples of any terms is illustrative only, and in no way limits the scope and meaning of the present invention or of any exemplified term. Likewise, the present invention is not limited to various embodiments given herein. Numbering terms such as "first," "second" or "third" can be used to describe various components, signals or the like, which are for distinguishing one component/signal from another one only, and are not intended to, nor should be construed to impose any substantive limitations on the components, signals or the like.

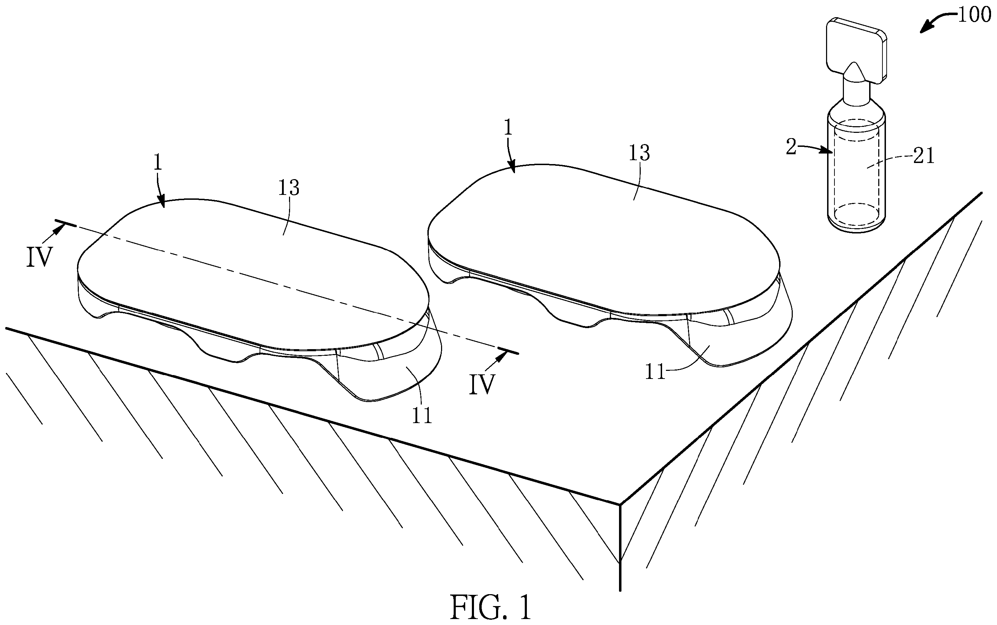

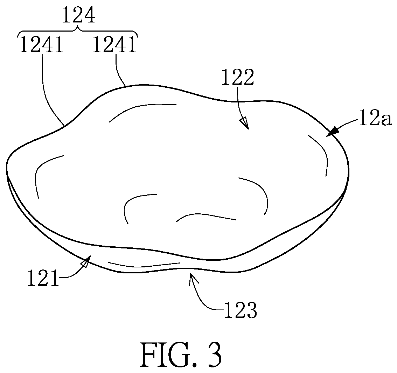

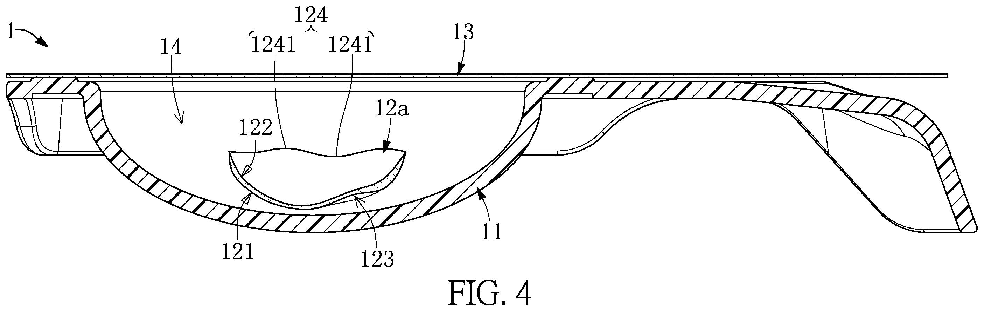

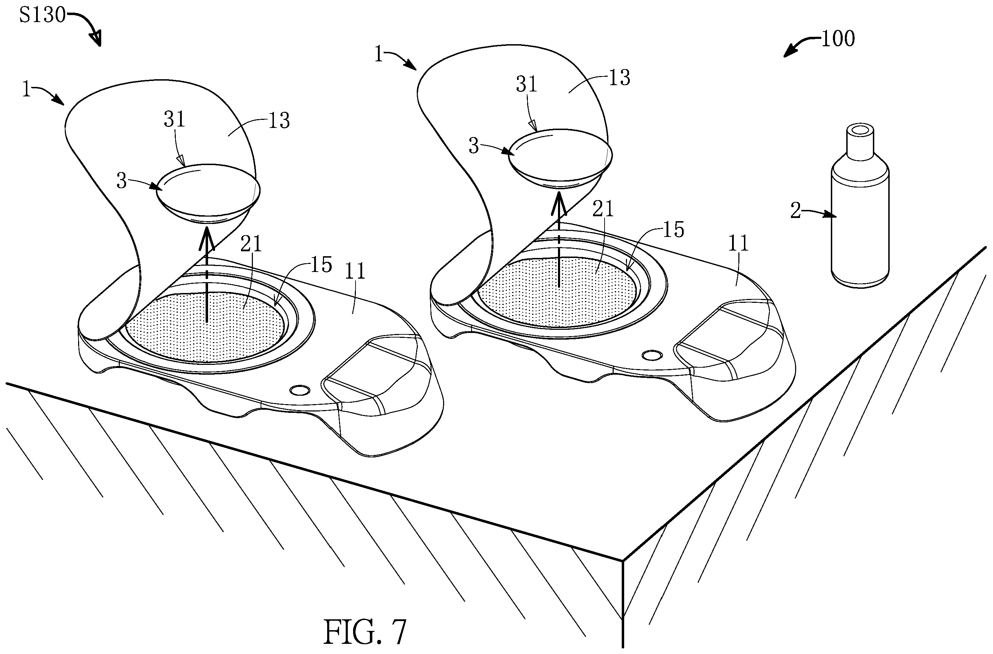

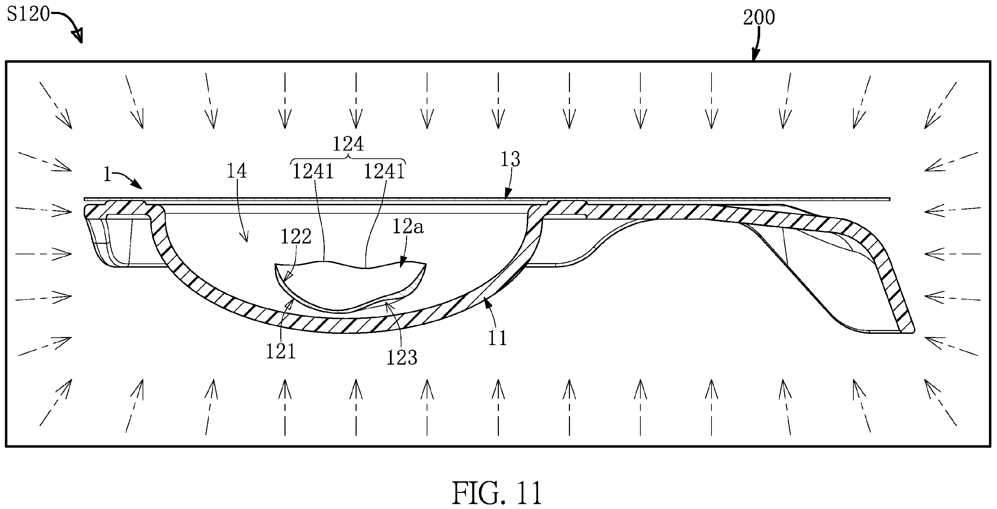

Referring to FIG. 1 to FIG. 12, an embodiment of the present invention is provided. The present embodiment provides a contact lens assembly 100 and a forming method S100 of a contact lens, and the forming method S100 is applied to the contact lens assembly 100, but the present invention is not limited thereto. In order to clearly describe the present embodiment, the following description describes the contact lens assembly 100, and then describes the forming method S100.

As shown in FIG. 1 to FIG. 4, the contact lens assembly 100 in the present embodiment includes a plurality of contact lens products 1 and a wetting liquid supply 2 that is in cooperation with any one of the contact lens products 1. It should be noted that the contact lens assembly 100 in the present embodiment is described by the cooperation between the contact lens products 1 and the wetting liquid supply 2, but the present invention is not limited thereto. For example, in other embodiments of the present invention not shown in the drawings, the contact lens assembly 100 can be provided with one of the contact lens products 1 and the wetting liquid supply 2 according to practical requirements; or, the contact lens product 1 can be independently used (e.g., sold) or can be used in cooperation with other components. In order to clearly describe the present embodiment, the following description describes just one of the contact lens products 1, and then describes the relationship of the contact lens products 1.

The contact lens product 1 includes a cup 11, a dry contact lens 12a, 12b disposed in the cup 11, and a sealing layer 13 that is removably connected to the cup 11 to jointly define an enclosed space 14. The enclosed space 14 receives the dry contact lens 12a, 12b and does not accommodate any liquid. In other words, the enclosed space 14 only allows the dry contact lens 12a, 12b and air to be located therein. Accordingly, any contact lens that is packed and immersed in liquid or that can be unpacked for immediate use is not the dry contact lens 12a, 12b provided by the present embodiment.

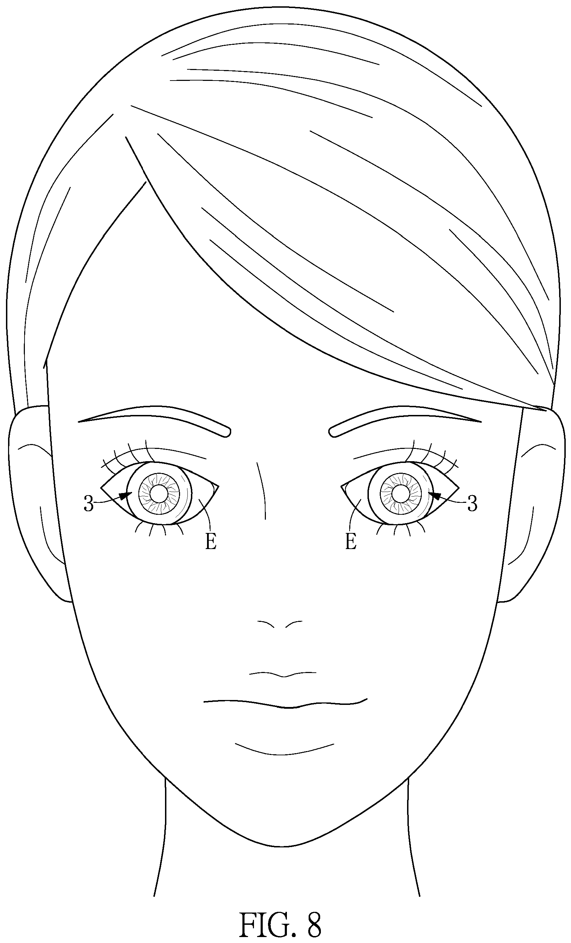

Specifically, the dry contact lens 12a, 12b has a shape (e.g., an irregular shape) that does not allow the dry contact lens 12a, 12b to be worn on a human eye E (as shown in FIG. 8), and the dry contact lens 12a, 12b has a first water content that can be within a range from 0.001% to 20% (e.g., a range from 3% to 10% being preferable), but the present invention is not limited thereto.

Moreover, the shape of the dry contact lens 12a, 12b is fixed and cannot be elastically deformable (e.g., the dry contact lens 12a, 12b cannot be flipped over and easily cracks under stress), and the dry contact lens 12a, 12b is free from cracks by having the first water content being greater than 0.001% (preferably greater than 3%). Specifically, the dry contact lens 12a, 12b has an outer surface 121 and an inner surface 122 that is opposite to the outer surface 121. The outer surface 121 of the dry contact lens 12a, 12b is arranged to face the cup 11, thereby reducing the possibility of the dry contact lens 12a, 12b being squeezed and broken when being placed in or taken out of the cup 11.

It should be noted that the shape of the dry contact lens 12a and 12b can be random irregular shapes (e.g., the shapes of the dry contact lenses 12a, 12b of the contact lens products 1 are of at least two kinds being different from each other), and the following description discloses shapes that are less likely to cause cracks, but the present invention is not limited thereto. In the present embodiment, the dry contact lens 12a can have a wrinkle 123 formed on the outer surface 121, and a peripheral edge 124 of the dry contact lens 12a, 12b can have a plurality of curved segments 1241 that have at least two radiuses of curvature different from each other.

In addition, the dry contact lens 12a, 12b in the present embodiment can be made of hydrogel, silicone hydrogel, or biodegradable material. The biodegradable material can be collagen or polysaccharide, but the present invention is not limited thereto.

The wetting liquid supply 2 has a wetting liquid 21 that is configured to wet the dry contact lens 12a, 12b, and the volume of the wetting liquid 21 contained in the wetting liquid supply 2 provided by the present embodiment is described as enabling the dry contact lenses 12a, 12b to be immersed therein, but the present invention is not limited thereto. For example, in other embodiments of the present invention not shown in the drawings, the volume of the wetting liquid 21 contained in the wetting liquid supply 2 can be provided to enable only a single dry contact lens 12a, 12b to be immersed therein according to practical requirements.

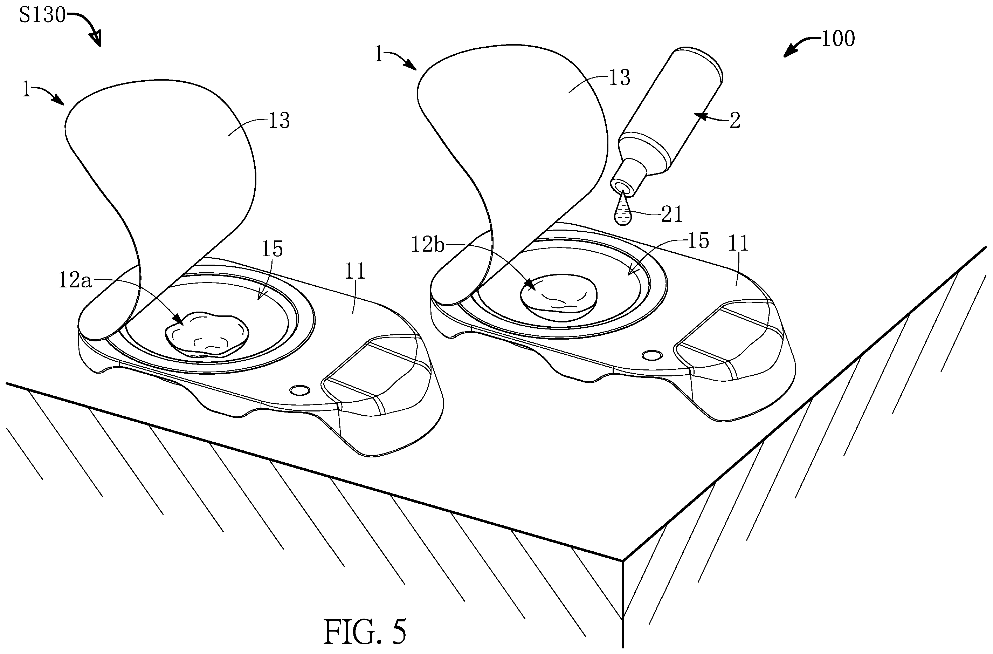

As shown in FIG. 5 to FIG. 8, when at least one of the sealing layer 13 and the cup 11 of any one of the contact lens products 1 produces an opening 15, the cup 11 is configured to receive the wetting liquid 21 from the wetting liquid supply 1 through the opening 15, so that the dry contact lens 12a, 12b is immersed in the wetting liquid 21 to become a wet contact lens 3 that has no cracks and that is configured to be worn on the human eye E, and the wet contact lens 3 has a second water content being greater than the first water content.

Specifically, after the dry contact lenses 12a, 12b having different shapes are immersed in the wetting liquid 21 to become the wet contact lenses 3, each of the wet contact lenses 3 does not have the wrinkle 123, and a peripheral edge of each of the wet contact lenses 3 has a circular shape. In other words, the wet contact lenses 3 have a same shape, but the present invention is not limited thereto. For example, in other embodiments of the present invention not shown in the drawings, the wet contact lenses 3 can be different from each other according to practical requirements (e.g., requirements for different diopters). Moreover, each of the wet contact lenses 3 has an elastically deformable shape (e.g., each of the wet contact lenses 3 can be flipped over), and the second water content of the wet contact lens 3 can be within a range from 24% to 79% (e.g., a range from 35% to 79% being preferable), but the present invention is not limited thereto.

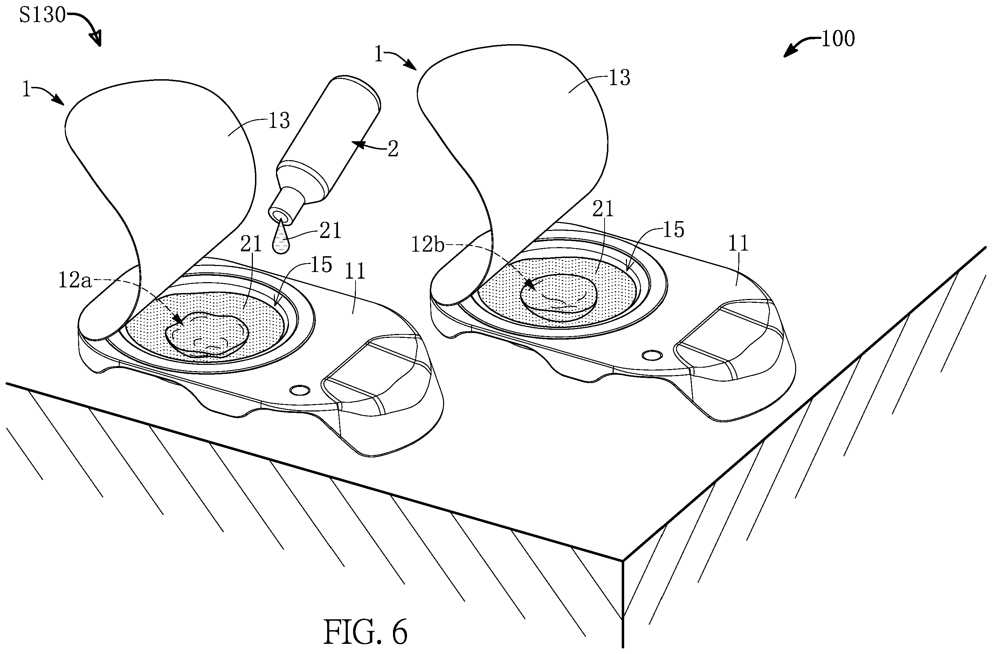

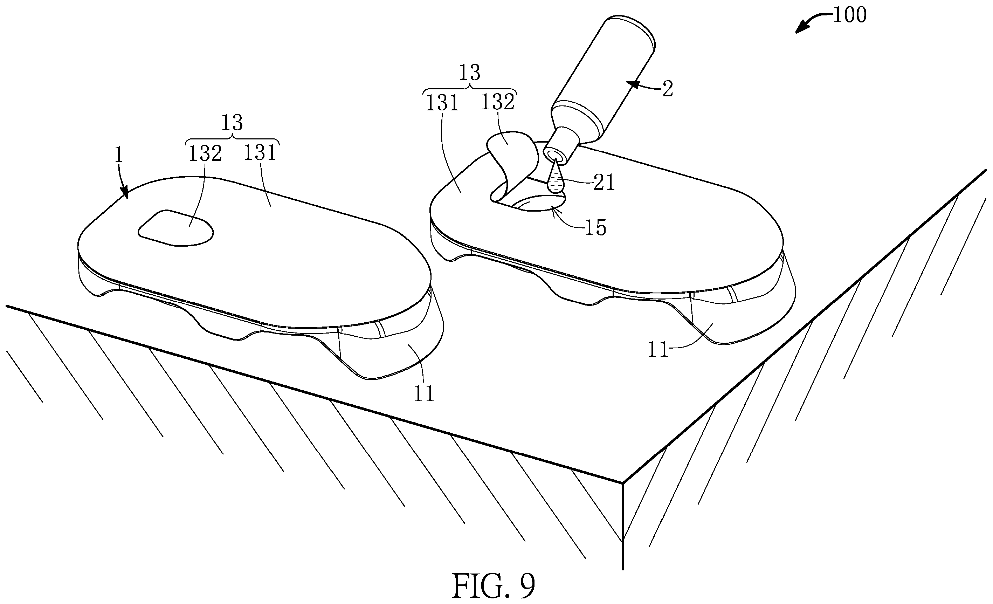

In addition, the opening 15 of the contact lens product 1 shown in FIG. 6 is formed by tearing away the sealing layer 13 from the cup 11, but the formation of the opening 15 can be adjusted or changed according to practical requirements. For example, as shown in FIG. 9, the sealing layer 13 can include a main sheet 131 connected to the cup 11 and a secondary sheet 132 that is connected to the main sheet 131 and that is substantially located above the enclosed space 14. The secondary sheet 132 can be torn from the main sheet 131 to form the opening 15 (e.g., the opening 15 can be formed only on the sealing layer 13 and corresponds in position to the enclosed space 14), such that after the cup 11 is filled with the wetting liquid 21 through the opening 15, the secondary sheet 132 can be adhered onto the main sheet 131 to enclose the opening 15, thereby reducing the possible external contamination or interference of the wetting liquid 21. Or, in other embodiments of the present invention not shown in the drawings, the opening 15 can be formed on the cup 11 and corresponds in position to the enclosed space 14.

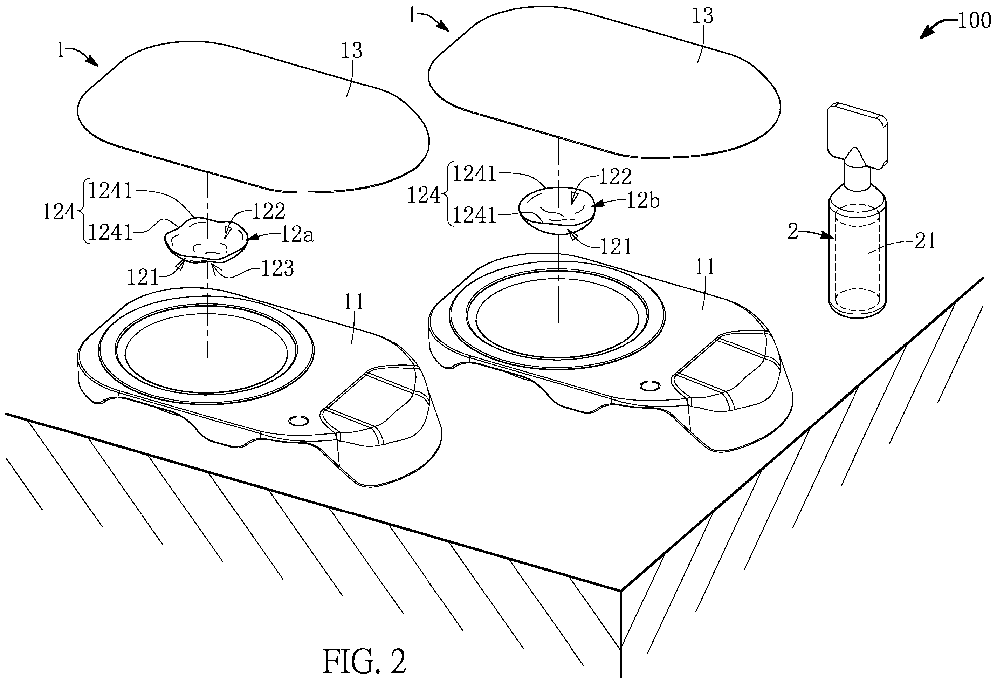

As shown in FIG. 2 to FIG. 7, in order for the wetting liquid 21 to facilitate the formation of the wet contact lens 3 by soaking the dry contact lens 12a and 12b, the wetting liquid 21 preferably has at least some of the following features, but the present invention is not limited thereto. The wetting liquid 21 has an osmolality that is within a range from 250 mOsmol/kg to 380 mOsmol/kg and a pH value that is within a range from 6.0 to 8.0. In other words, the wetting liquid 21 is preferably a solution that has an isotonicity and a pH value close to neutral.

Moreover, the wetting liquid 21 includes a saline buffer that can be a borate buffer or a phosphate buffer. The wetting liquid 21 can further include at least one of a hydrophilic polymer, a surfactant, and a functional additive.

In the present embodiment, the hydrophilic polymer can be at least one of (e.g., can be selected from the group consisting of) γ-polyglutamic acid sodium, hyaluronic acid, methacrylate phosphatidylcholine (MPC), hydroxypropyl methylcellulose (HPMC), polyethylene glycol, polysaccharides, sodium alginate, sodium hyaluronate, carboxymethyl cellulose, and polyvinyl alcohol. The surfactant can be at least one of (e.g., can be selected from the group consisting of) polysorbates, sulfosuccinate esters, polyoxypropylene glycols, sodium lauryl sulfate, Geropon SBFA-30, and sodium laureth sulfate.

In addition, the functional additive of the present embodiment includes antioxidant ingredients that are easily degraded at high temperatures, and the functional additive can be at least one of other ingredients that are easily degraded at high temperatures, cooling agents, and drug ingredients according to practical requirements, but the present invention is not limited thereto. For example, the drug ingredients can be water-soluble drugs or hydrophobic drugs. The water-soluble drugs can be at least one of diclofenac, timolol, pilocarpine, atropine, and brimonidine. Moreover, the hydrophobic drugs can be at least one of dexamethasone, axitinib, sunitinib, and cyclosporine.

Specifically, the wetting liquid 21 is provided after being sterilized through a liquid sterilization process, and the antioxidant component contained in the wetting liquid 21 is reduced from a pre-sterilization concentration to a post-sterilization concentration that is at least 70% of the pre-sterilization concentration. The antioxidant component can include at least one of vitamin E, vitamin C, carotenoids, lutein, zeaxanthin, alpha-lipoic acid, anthocyanin-rich grape seed extract, catechin-rich green tea extract, resveratrol, and squalene.

In summary, the contact lens assembly 100 (and the contact lens product 1) in the present embodiment can be provided with a configuration (e.g., the dry contact lens 12a, 12b arranged in the enclosed space 14 defined by the cup 11 and the sealing layer 13) different from that of the conventional contact lens product, thereby allowing the scope of development for contact lenses to be expanded (e.g., allowing the dry contact lens 12a, 12b and the wetting liquid 21 to be sterilized independently to improve the overall performance).

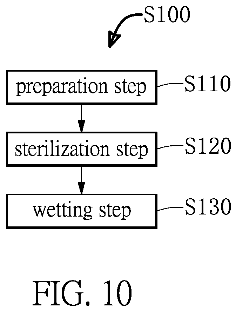

The above description substantially describes the configuration and corresponding effects of the contact lens assembly 100, and the following description describes the forming method S100 (shown in FIG. 10) that is applied to the contact lens assembly 100. Moreover, the relevant technical content of the forming method S100 can refer to the above description of the contact lens assembly 100 (e.g., the structure and composition of the dry contact lens 12a, 12b, the composition of the wetting liquid 21, and the structure and composition of the wet contact lens 3) for the sake of brevity.

As shown in FIG. 10, the forming method S100 in the present embodiment includes a preparation step S 110, a sterilization step S 120, and a wetting step S 130, and the following description substantially describes each of the above steps. Each of the above steps can be adjusted according to practical requirements, and the present invention is not limited thereto.

As shown in FIG. 10, FIG. 1, FIG. 2, and FIG. 4, the preparation step S 110 is implemented by placing the dry contact lens 12a, 12b that has the first water content into the cup 11 after a drying process, and then using the sealing layer 13 to seal the cup 11 to jointly form the enclosed space 14 that receives the dry contact lens 12a, 12b and that does not accommodate any liquid.

In the present embodiment, the dry contact lens 12a, 12b is provided by performing a hydration process and a swelling process before the drying process. In other words, the dry contact lens 12a, 12b is cleaned and removed of impurities before the drying process to avoid the presence of substances that irritate the human eye E (shown in FIG. 8). Moreover, the drying process can be varied according to practical requirements, such as a natural drying process, an oven heating drying process (e.g., an oven temperature being within a range from 30°C to 80°C), or a freeze drying process, so that the first water content of the dry contact lens 12a, 12b after the drying process can be within a range from 0.001% to 20% (e.g., a range from 3% to 10% being preferable), but the present invention is not limited thereto.

As shown in FIG. 10, FIG. 2, and FIG. 11, the sterilization step S120 is implemented by performing a dry contact lens sterilization process for the dry contact lens 12a, 12b that is in the enclosed space 14. In other words, the cup 11, the sealing layer 13, and the dry contact lens 12a, 12b are jointly defined as the contact lens product 1 after the dry lens sterilization process.

In the sterilization step S120, the dry contact lens 12a, 12b is not immersed in any liquid, so that the implementation and environmental parameters of the dry lens sterilization process can be selected and adjusted for the dry contact lens 12a, 12b (e.g., various dry contact lenses 12a, 12b made of different materials may be suited to different sterilization processes and parameters), thereby achieving a better sterilization effect for the dry contact lens 12a, 12b.

For example, the dry contact lens 12a, 12b in the present embodiment can reach a sterility assurance level (SAL) of 10⁻⁶. The dry lens sterilization process can be a radiation sterilization process that can be implemented by a radiation sterilization device 200, an ethylene oxide (EO) sterilization process, or an autoclaved process according to practical requirements, but the present invention is not limited thereto.

As shown in FIG. 10 and FIG. 5 to FIG. 8, the wetting step S130 is implemented by tearing away the sealing layer 13 relative to the cup 11 to form the opening 15, and then injecting the wetting liquid 21 into the cup 11 through the opening 15, such that after the dry contact lens 12a, 12b is immersed in the wetting liquid 21 for a predetermined period of time, the dry contact lens 12a, 12b becomes the wet contact lens 3 that is configured to be worn on the human eye E and that has the second water content greater than the first water content.

In the present embodiment, the wetting liquid 21 has the osmolality that is within a range from 250 mOsmol/kg to 380 mOsmol/kg and the pH value that is within a range from 6.0 to 8.0, so that the wetting liquid 21 is suitable for forming the dry contact lens 12a, 12b into the wet contact lens 3. Moreover, the predetermined period of time is at least 5 minutes (e.g., within a range from 5 minutes to 15 minutes), and the second water content of the wet contact lens 3 is within a range from 24% to 79% (e.g., a range from 35% to 79% being preferable).

In addition, after the physical dimensions of the wet contact lens 3 (e.g., diameter, base curve, diopter, and thickness) are inspected in accordance with ISO 18369-3:2017 specifications, the wet contact lens 3 fully complies with the specifications and has no cracks, meeting the relevant safety requirements for being directly worn on the human eye E.

Before the wetting step S130 is implemented, the wetting liquid 21 is sterilized through a liquid sterilization process, and the antioxidant component contained in the wetting liquid 21 is reduced from the pre-sterilization concentration to the post-sterilization concentration that is at least 70% of the pre-sterilization concentration. Furthermore, the implementation and environmental parameters of the liquid sterilization process can be selected and adjusted for the wetting liquid 21, thereby achieving a better sterilization effect for the wetting liquid 21.

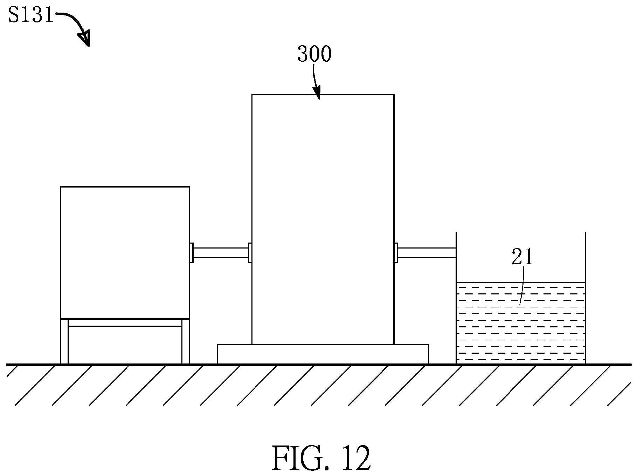

The liquid sterilization process in the present embodiment can be performed according to practical requirements by using a sterile filtration process S131 (shown in FIG. 12), a radiation sterilization process, or an ethylene oxide sterilization process, so that the wetting liquid 21 can reach a sterility assurance level of 10⁻⁶. For example, when the antioxidant ingredients are vitamin C, after the sterile filtration process S131 (shown in FIG. 12) is performed to the wetting liquid 21 through the sterile filtration apparatus 300, the post-sterilization concentration can reach at least 90% (e.g., 98%) of the pre-sterilization concentration, thereby being significantly better than that in the relevant art, in which the post-sterilization concentration of the antioxidant ingredients (e.g., vitamin C) of the maintenance liquid remains less than 10% of the pre-sterilization concentration after the contact lens and the maintenance liquid are sterilized simultaneously.

In other words, the dry contact lens 12a, 12b and the wetting liquid 21 in the present embodiment are sterilized by respectively performing independent sterilization processes (e.g., the dry lens sterilization process and the liquid sterilization process), such that under the premise of meeting their respective sterilization requirements, the dry contact lens 12a, 12b and the wetting liquid 21 can further effectively retain their optimal performance (e.g., the dry contact lens 12a, 12b made of biodegradable materials and the ingredients contained in the wetting liquid 21 that are easily degraded at high temperatures can effectively avoid being reduced).

Moreover, since the wetting liquid 21 has a high content of the functional additives (e.g., the antioxidant ingredients), the wet contact lenses 3 formed by using the dry contact lenses 12a, 12b to be immersed in the wetting liquid 21 can effectively absorb the functional additives and have better performance.

Furthermore, after the dry contact lens 12a, 12b and the wetting liquid 21 are subjected to sterility testing for aerobic, anaerobic, and fungal species by a third party agency (e.g., SGS) in accordance with USP<71> specifications, the dry contact lens 12a, 12b and the wetting liquid 21 are ensured to be in a sterile state.

In addition, since the dry sheet sterilization process and the liquid sterilization process are performed independently, the liquid sterilization process can be performed before the sterilization step S 120, after the sterilization step S 120, or simultaneously with the sterilization step S 120 according to practical requirements, and the present invention is not limited thereto.

### [Beneficial Effects of the Embodiment]

In conclusion, any one of the forming method, the contact lens assembly, and the contact lens product in the present invention is provided with a configuration (e.g., the dry contact lens arranged in the enclosed space defined by the cup and the sealing layer) different from that of the conventional contact lens product, thereby allowing the scope of development for contact lenses to be expanded (e.g., allowing the dry contact lens and the wetting liquid to be sterilized independently to improve the overall performance).

Specifically, in any one of the forming method and the contact lens assembly provided by the present invention, the dry contact lens and the wetting liquid are sterilized by respectively performing independent sterilization processes, such that under the premise of meeting their respective sterilization requirements, the dry contact lens and the wetting liquid can further effectively retain their optimal performance.

The foregoing description of the exemplary embodiments of the invention has been presented only for the purposes of illustration and description and is not intended to be exhaustive or to limit the invention to the precise forms disclosed. Many modifications and variations are possible in light of the above teaching.

The embodiments were chosen and described in order to explain the principles of the invention and their practical application so as to enable others skilled in the art to utilize the invention and various embodiments and with various modifications as are suited to the particular use contemplated.

## Claims

1. A forming method (S100) of a contact lens, **characterized by** comprising:
a preparation step (S110) implemented by placing a dry contact lens (12a, 12b) that has a first water content into a cup (11) after a drying process, and then using a sealing layer (13) to seal the cup (11) for jointly forming an enclosed space (14) that receives the dry contact lens (12a, 12b) and that does not accommodate any liquid;
a sterilization step (S120) implemented by performing a dry contact lens sterilization process for the dry contact lens (12a, 12b) that is in the enclosed space (14); and
a wetting step (S130) implemented by tearing away the sealing layer (13) relative to the cup (11) to form an opening (15), and then injecting a wetting liquid (21) into the cup (11) through the opening (15), wherein, after the dry contact lens (12a, 12b) is immersed in the wetting liquid (21) for a predetermined period of time, the dry contact lens (12a, 12b) becomes a wet contact lens (3) that is configured to be worn on a human eye (E) and that has a second water content greater than the first water content.

2. The forming method (S100) according to claim 1, wherein, before the wetting step (S130) is implemented, the wetting liquid (21) is sterilized through a liquid sterilization process, and an antioxidant component contained in the wetting liquid (21) is reduced from a pre-sterilization concentration to a post-sterilization concentration that is at least 70% of the pre-sterilization concentration.

3. The forming method (S100) according to claim 2, wherein the wetting liquid (21) has an osmolality that is within a range from 250 mOsmol/kg to 380 mOsmol/kg and a pH value that is within a range from 6.0 to 8.0.

4. The forming method (S100) according to claim 2, wherein the antioxidant component includes at least one of vitamin E, vitamin C, carotenoids, lutein, zeaxanthin, alpha-lipoic acid, anthocyanin-rich grape seed extract, catechin-rich green tea extract, resveratrol, and squalene.

5. The forming method (S100) according to claim 2, wherein the liquid sterilization process is a sterile filtration process (S131), a radiation sterilization process, or an ethylene oxide sterilization process.

6. The forming method (S100) according to claim 1, wherein, in the preparation step (S110), the first water content of the dry contact lens (12a, 12b) is within a range from 0.001% to 20%, and wherein, in the wetting step (S130), the predetermined period of time is at least 5 minutes, and the second water content of the wet contact lens (3) is within a range from 24% to 79%.

7. The forming method (S100) according to claim 1, wherein the dry contact lens sterilization process is a radiation sterilization process, an ethylene oxide sterilization process, or an autoclaved process.

8. A contact lens assembly (100), **characterized by** comprising:
a contact lens product (1) including:
a cup (11);
a dry contact lens (12a, 12b) disposed in the cup (11) and having a first water content, wherein the dry contact lens (12a, 12b) has a shape that does not allow the dry contact lens (12a, 12b) to be worn on a human eye (E); and
a sealing layer (13) that is removably connected to the cup (11) to jointly define an enclosed space (14), wherein the enclosed space (14) receives the dry contact lens (12a, 12b) and does not accommodate any liquid; and
a wetting liquid supply (2) having a wetting liquid (21) that is configured to wet the dry contact lens (12a, 12b);
wherein, when at least one of the sealing layer (13) and the cup (11) of the contact lens product (1) produces an opening (15), the cup (11) is configured to receive the wetting liquid (21) from the wetting liquid supply (2) through the opening (15), so that the dry contact lens (12a, 12b) is immersed in the wetting liquid (21) to become a wet contact lens (3) that has no cracks and that is configured to be worn on the human eye (E), and wherein the wet contact lens (3) has a second water content being greater than the first water content.

9. The contact lens assembly (100) according to claim 8, wherein the first water content of the dry contact lens (12a, 12b) is within a range from 0.001% to 20%, and the second water content of the wet contact lens (3) is within a range from 24% to 79%.

10. The contact lens assembly (100) according to claim 8, wherein the first water content of the dry contact lens (12a, 12b) is within a range from 3% to 10%, and the second water content of the wet contact lens (3) is within a range from 35% to 79%.

11. The contact lens assembly (100) according to claim 8, wherein the dry contact lens (12a, 12b) has an outer surface (121) facing toward the cup (11) and an inner surface (122) that is opposite to the outer surface (121), and wherein the dry contact lens (12a, 12b) has a wrinkle (123) formed on the outer surface (121), and the wet contact lens (3) does not have the wrinkle (123).

12. The contact lens assembly (100) according to claim 8, wherein a peripheral edge (124) of the dry contact lens (12a, 12b) has a plurality of curved segments (1241) that have at least two radiuses of curvature different from each other, and a peripheral edge (31) of the wet contact lens (3) has a circular shape.

13. The contact lens assembly (100) according to claim 8, wherein the shape of the dry contact lens (12a, 12b) is fixed, and the wet contact lens (3) has an elastically deformable shape.

14. The contact lens assembly (100) according to claim 8, wherein the wetting liquid (21) has an osmolality that is within a range from 250 mOsmol/kg to 380 mOsmol/kg and a pH value that is within a range from 6.0 to 8.0.

15. The contact lens assembly (100) according to claim 8, wherein the wetting liquid (21) is provided after being sterilized through a liquid sterilization process, and an antioxidant component contained in the wetting liquid (21) is reduced from a pre-sterilization concentration to a post-sterilization concentration that is at least 70% of the pre-sterilization concentration.

16. The contact lens assembly (100) according to claim 15, wherein the antioxidant component includes at least one of vitamin E, vitamin C, carotenoids, lutein, zeaxanthin, alpha-lipoic acid, anthocyanin-rich grape seed extract, catechin-rich green tea extract, resveratrol, and squalene.

17. The contact lens assembly (100) according to claim 8, further comprising a plurality of contact lens products (1), wherein the shapes of the dry contact lenses (12a, 12b) of the contact lens products (1) are of at least two kinds being different from each other, and the wet contact lenses (3) have a same shape.

18. A contact lens product (1), **characterized by** comprising:
a cup (11);
a dry contact lens (12a, 12b) disposed in the cup (11) and having a first water content that is within a range from 0.001% to 20%, wherein the dry contact lens (12a, 12b) has a shape that does not allow the dry contact lens (12a, 12b) to be worn on a human eye (E); and
a sealing layer (13) that is removably connected to the cup (11) to jointly define an enclosed space (14), wherein the enclosed space (14) receives the dry contact lens (12a, 12b) and does not accommodate any liquid.

19. The contact lens product (1) according to claim 18, wherein the first water content of the dry contact lens (12a, 12b) is within a range from 3% to 10%, and the shape of the dry contact lens (12a, 12b) is fixed, and wherein a peripheral edge (124) of the dry contact lens (12a, 12b) has a plurality of curved segments (1241) that have at least two radiuses of curvature different from each other.

20. The contact lens product (1) according to claim 18, wherein the first water content of the dry contact lens (12a, 12b) is within a range from 3% to 10%, and the shape of the dry contact lens (12a, 12b) is fixed, and wherein the dry contact lens (12a, 12b) has an outer surface (121) facing toward the cup (11) and an inner surface (122) that is opposite to the outer surface (121), and the dry contact lens (12a, 12b) has a wrinkle (123) formed on the outer surface (121).
